(19) Europäisches Patentamt
European Patent Office
Office européen des brevets

(11) EP 4 212 502 A1

(12) EUROPÄISCHE PATENTANMELDUNG

(43) Veröffentlichungstag:
19.07.2023 Patentblatt 2023/29

(21) Anmeldenummer: 22151780.8

(22) Anmeldetag: 17.01.2022

(51) Internationale Patentklassifikation (IPC):
C07C 29/152 (2006.01)    C07C 31/04 (2006.01)
C01B 3/34 (2006.01)      B01J 8/00 (2006.01)

(52) Gemeinsame Patentklassifikation (CPC):
(C-Sets verfügbar)
B01J 8/00; C01B 3/34; C07C 29/152        (Forts.)

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(71) Anmelder: GasConTec GmbH
61348 Bad Homburg v. d. Höhe (DE)

(72) Erfinder:
• LAUER, Wolfgang
34613 Schwalmstat (DE)
• MÜLLER, Dierk
61184 Karben (DE)
• RAPPOLD, Dorit
60437 Frankfurt (DE)
• WAGNER, Ulrich
06406 Bernburg (DE)

(74) Vertreter: Patentanwälte Bauer Vorberg Kayser
Partnerschaft mbB
Goltsteinstraße 87
50968 Köln (DE)

(54) **VERFAHREN UND ANLAGE ZUR SYNTHESE VON METHANOL**

(57) Die Erfindung betrifft ein Verfahren zur Synthese von Methanol (1), wobei ein Synthesegasstrom (2) mit Wasserstoff und Kohlenstoffoxiden einer Methanolreaktoranordnung (3) zur Synthese von Methanol (1) zugeführt wird, wobei ein Restgasstrom (4) mit unreagiertem Wasserstoff und unreagierten Kohlenstoffoxiden aus der Methanolreaktoranordnung (3) gewonnen wird, wobei der Restgasstrom (4) einer Wasserstoffabtrennungsanordnung (5) zum Erhalt eines wasserstoffhaltigen Stroms (6) zugeführt, wobei der wasserstoffhaltige Strom (6) der Methanolreaktoranordnung (3) zugeführt wird und einen höheren molaren Wasserstoffanteil als der Restgasstrom (4) aufweist und wobei ein Purgegasstrom (7) mit Methan aus der Wasserstoffabtrennungsanordnung (5) gewonnen wird, dadurch gekennzeichnet, dass der Purgegasstrom (7) einer Rückgewinnungsanordnung (8) zum Gewinnen eines Rückgewinnungsstroms (9) mit Kohlenstoffdioxid und Wasserstoff zugeführt wird, dass die Rückgewinnungsanordnung (8) einen Rückgewinnungsreaktor (10) zum zumindest teilweisen Umwandeln des Methans des Purgegasstroms (7) in Kohlenstoffoxide und Wasserstoff durch thermische partielle Oxidation aufweist und dass der Rückgewinnungsstrom (9) der Methanolreaktoranordnung (3) zugeführt wird. Ebenso betrifft die Erfindung eine entsprechende Anlage zur Synthese von Methanol.

Fig. 1

**(Forts. nächste Seite)**

(52) Gemeinsame Patentklassifikation (CPC): (Forts.)

C-Sets
**C07C 29/152, C07C 31/04**

**Beschreibung**

Die Erfindung betrifft ein Verfahren zur Synthese von Methanol gemäß dem Oberbegriff von Patentanspruch 1 und eine Anlage zur Synthese von Methanol gemäß dem Oberbegriff von Patentanspruch 15.

Ein zunehmend wichtiger Aspekt bei der Methanolsynthese ist die Verringerung oder Vermeidung der Emission speziell von Kohlenstoffdioxid im Rahmen der Synthese von Methanol. Auch aus dem Stand der Technik ergeben sich je nach Art der für die Methanolsynthese verwendeten Technologie wesentliche Unterschiede. So weist eine mit einem autothermen Reformer (ATR) zur Erzeugung von Synthesegas betriebene Methanolanlage, wie sie etwa in der als nächstkommend angesehenen EP 3 205 622 B1 beschrieben wird, im Vergleich zu anderen Arten von Methanolanlagen, etwa mit einem Steam Reformer zur Synthesegaserzeugung, nur 40 % der Emissionen an Kohlenstoffdioxid auf.

Die wesentlichen Ursachen für diese, wie festgestellt bereits vergleichsweise geringen Emissionen von Kohlenstoffdioxid, sind die alleinige Verfeuerung von Erdgas und Purgegas für den Betrieb von Wärmetauschern zur Dampfüberhitzung und zur Erdgasvorwärmung sowie das Erfordernis, zusätzlichen Wasserstoff zum Ausgleich des bei der autothermen Reformierung regelmäßig unterstöchiometrischen Synthesegases einzubringen. Die Dampfüberhitzung wird regelmäßig zum Betrieb von Turbinen benötigt, welche wiederum die Kompressoren antreiben.

Im Lichte dieses Umstands ließen sich grundsätzlich also auch die verbliebenen Emissionen an Kohlenstoffdioxid weitestgehend dadurch vermeiden, dass erstens die Wärmetauscher elektrisch, und zwar unter Verwendung klimaneutraler Energiequellen, betrieben werden und dass zweitens auch eine zusätzliche Wasserelektrolyse zum Gewinnen des besagten Wasserstoffs mit Strom aus klimaneutralen Energiequellen durchgeführt wird.

Jedoch bleibt, wenn man diese Maßnahmen umsetzt, das Problem, was nunmehr mit dem nun nicht mehr in den Wärmetauschern zu verbrennendem Purgegas geschehen soll. Einerseits ergibt sich aus dem zum Teil erheblichen Stickstoffanteil bereits im Frischgas die Notwendigkeit, den Stickstoff aus dem Kreislauf der Methanolsynthese abzuführen, da ansonsten das Gesamtvolumen an Gas im Synthesekreislauf zu sehr ansteigt, was wiederum eine größere Dimensionierung der Anlage und insbesondere der energieintensiven Kompressoren erfordert bzw. bei absolutem Nichtabscheiden bis zum Ausfall der Anlage führt. Andererseits ist der Gehalt sowohl von Kohlenstoffdioxid als auch von Methan im Purgegas zu hoch, als dass das Purgegas einfach verfeuert oder anderweitig in die Atmosphäre abgegeben werden könnte, ohne die gewünschte Verringerung der Kohlenstoffdioxidemissionen zu verfehlen.

Ausgehend von diesem Hintergrund besteht die Aufgabe der Erfindung daher darin, einen Ansatz für die Methanolsynthese bereitzustellen, welcher einen wirtschaftlichen Betrieb weitgehend frei von Kohlenstoffdioxidemissionen erlaubt, wobei das nicht mehr emittierte Kohlenstoffdioxid in Methanol überführt wird.

Bezogen auf ein Verfahren zur Synthese von Methanol mit den Merkmalen des Oberbegriffs von Anspruch 1 wird diese Aufgabe durch die Merkmale des kennzeichnenden Teils von Anspruch 1 gelöst. Bezogen auf eine Anlage zur Synthese von Methanol mit den Merkmalen des Oberbegriffs von Anspruch 15 wird diese Aufgabe durch die Merkmale des kennzeichnenden Teils von Anspruch 15 gelöst.

Der Erfindung liegt die Erkenntnis zugrunde, dass ein aus dem Synthesekreislauf entfernter Purgegasstrom zum Zweck der Kohlenstoffdioxidneutralität dadurch aufbereitet werden kann, dass dieser Purgegasstrom einem Reaktor zur thermischen partiellen Oxidation zugeführt wird. Denn mittels thermischer partieller Oxidation wird erreicht, dass das in dem Purgegasstrom enthaltene Methan in Kohlenstoffoxide und Wasserstoff umgewandelt wird. Auf diese Weise wird anschließend eine separate Abtrennung sowohl der Kohlenstoffoxide als auch des Wasserstoffs aus dem Purgegasstrom möglich. Regelmäßig verbleiben nach dieser Abtrennung dann nur noch Stickstoff und Edelgasanteile in einem Restgasstrom, sodass dieser Restgasstrom sowohl ohne klimabelastendes Kohlenstoffdioxid als auch ohne für die Methanolsynthese an sich wertvollem Wasserstoff in die Umgebung abgegeben werden kann. Das abgetrennte Kohlenstoffdioxid und der abgetrennte Wasserstoff wiederum können in den Kreislauf der Methanolsynthese zurückgeführt werden und ermöglichen damit die Umwandlung des ansonsten emittierten Kohlenstoffdioxids in Methanol.

Der Unteranspruch 2 beschreibt eine vorteilhafte Möglichkeit, das Kohlenstoffmonoxid aus dem Purgegasstrom nach der partiellen Oxidation in Kohlenstoffdioxid umzuwandeln, sodass dieses einfach aus dem Purgegasstrom entfernt werden kann. Der Unteranspruch 3 wiederum beschreibt eine vorteilhafte Möglichkeit zum möglichst vollständigen Entfernen des Kohlenstoffdioxids aus dem von Kohlenstoffmonoxid befreiten Purgegas.

Die Unteransprüche 4, 5 und 6 wiederum betreffen bevorzugte Varianten zur möglichst vollständigen Wiedergewinnung von Wasserstoff aus dem Purgegasstrom.

Eine kohlenstoffdioxidneutrale Möglichkeit des Betriebs der Wärmetauscher wird im Unteranspruch 8 und entsprechende Möglichkeiten des Betriebs eines Synthesegaskompressors werden in den Unteransprüchen 10 und 11 beschrieben.

Eine gerade für unterstöchiometrisches Synthesegas effiziente Reaktorausgestaltung für die Methanolsynthese ist Gegenstand des Unteranspruchs 13.

Schließlich sieht der Gegenstand des Unteranspruchs 14 die Zuführung von insbesondere klimaneutral gewonnenem Wasserstoff zur Anpassung der Stöchiometrie vor.

Bevorzugte Ausgestaltungen, Merkmale und Vorteile des erfindungsgemäßen Verfahrens zur Synthese von Methanol entsprechen bevorzugten Ausgestaltungen, Merkmalen und Vorteilen der erfindungsgemäßen Anlage zur Synthese von Methanol und umgekehrt.

Weitere Einzelheiten, Merkmale, Ziele und Vorteile der vorliegenden Erfindung werden nachfolgend anhand einer lediglich ein Ausführungsbeispiel wiedergebenden Zeichnung erläutert. In der Zeichnung zeigt

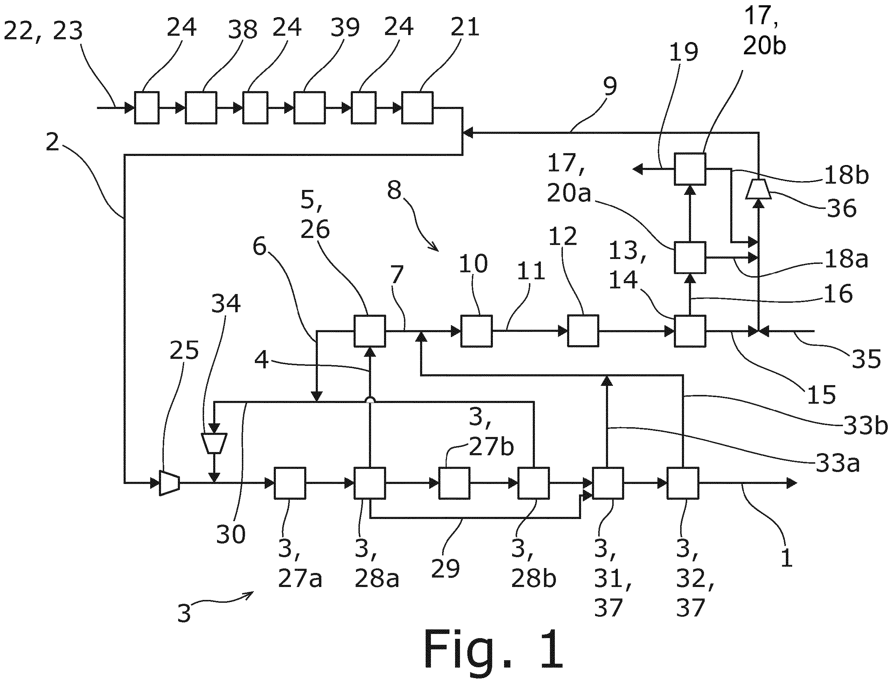

Fig. 1    eine schematische Darstellung eines Ausführungsbeispiels der vorschlagsgemäßen Anlage zur Synthese von Methanol zur Ausführung des vorschlagsgemäßen Verfahrens zur Synthese von Methanol.

Das vorschlagsgemäße Verfahren zur Synthese von Methanol 1 kann durch die vorschlagsgemäße Anlage zur Synthese von Methanol 1 gemäß dem Ausführungsbeispiel der Fig. 1 durchgeführt werden.

Beim vorschlagsgemäßen Verfahren zur Synthese von Methanol 1 wird ein Synthesegasstrom 2 mit Wasserstoff und Kohlenstoffoxiden einer Methanolreaktoranordnung 3 der vorschlagsgemäßen Anlage zur Synthese von Methanol 1 zugeführt. Insbesondere kann es sein, dass der Synthesegasstrom 2 einen molaren Anteil an Wasserstoff von mindestens 50 % oder mindestens 60 %, alternativ oder zusätzlich einen molaren Anteil an Kohlenstoffdioxid von mindestens 5 % und/oder höchstens 10 %, alternativ oder zusätzlich einen molaren Anteil an Kohlenstoffmonoxid von mindestens 15 % und/oder höchstens 30 % sowie alternativ oder zusätzlich einen molaren Anteil an Methan von mindestens 1 % und/oder höchstens 5 % aufweist.

Weiter wird bei dem vorschlagsgemäßen Verfahren aus der Methanolreaktoranordnung 3 ein Restgasstrom 4 mit unreagiertem Wasserstoff und unreagierten Kohlenstoffoxiden gewonnen, welcher Restgasstrom 4 einer Wasserstoffabtrennungsanordnung 5 der vorschlagsgemäßen Anlage zum Erhalt eines wasserstoffhaltigen Stroms 6 zugeführt wird, wobei der wasserstoffhaltige Strom 6 der Methanolreaktoranordnung 3 zugeführt wird und einen höheren molaren Wasserstoffanteil als der Restgasstrom 4 aufweist. Bevorzugt ist hier, dass der Restgasstrom 4 einen molaren Wasserstoffanteil von mindestens 60 % und insbesondere von mindestens 70 % aufweist. Vorzugsweise beträgt der molare Anteil von Kohlenstoffoxiden im Restgasstrom 4 mindestens 5 % und/oder höchstens 15 %. Der molare Anteil von Methan im Restgasstrom 4 beträgt vorzugsweise mindestens 10 % und/oder höchstens 25 %. Der molare Anteil von Stickstoff im Restgasstrom 4 beträgt vorzugsweise höchstens 10 %.

Schließlich wird bei dem vorschlagsgemäßen Verfahren ein Purgegasstrom 7 mit Methan aus der Wasserstoffabtrennungsanordnung 5 gewonnen. Neben Methan kann der Purgegasstrom 7 weitere Bestandteile aufweisen. Es kann sein, dass der Purgegasstrom 7 Kohlenstoffdioxid, Kohlenstoffmonoxid, Stickstoff und Argon aufweist und insbesondere aus diesen Bestandteilen besteht. Vorzugsweise umfasst der Purgegasstrom 7 einen molaren Anteil an Wasserstoff von höchstens 50 %, insbesondere von höchstens 40 %. Ebenso bevorzugt ist, dass der kombinierte molare Anteil von Kohlenstoffdioxid und Kohlenstoffmonoxid in dem Purgegasstrom 7 höchstens 30 % beträgt. Es kann sein, dass der Purgegasstrom 7 einen molaren Anteil an Methan von mindestens 15 % oder von mindestens 20 % aufweist. Schließlich kann es sein, dass der Purgegasstrom 7 einen molaren Anteil an Stickstoff von mindestens 5 % und/oder von höchstens 30 % aufweist. Der molare Anteil an Argon im Purgegasstrom 7 beträgt vorzugsweise mindestens 0,1 % und höchstens 5 %.

Das vorschlagsgemäße Verfahren ist dadurch gekennzeichnet, dass der Purgegasstrom 7 einer Rückgewinnungsanordnung 8 der vorschlagsgemäßen Anlage zum Gewinnen eines Rückgewinnungsstroms 9 mit Kohlenstoffdioxid und Wasserstoff zugeführt wird, dass die Rückgewinnungsanordnung 8 einen Rückgewinnungsreaktor 10 zum zumindest teilweisen, vorzugsweise im Wesentlichen vollständigen, Umwandeln des Methans des Purgegasstroms 7 in Kohlenstoffoxide und Wasserstoff durch thermische partielle Oxidation aufweist und dass der Rückgewinnungsstrom 9 der Methanolreaktoranordnung 3 zugeführt wird. Der Rückgewinnungsstrom 9 kann speziell aus dem Rückgewinnungsreaktor 10 gewonnen werden. Es kann aber auch sein, dass der Rückgewinnungsstrom 9 - wie untenstehend beschrieben - aus einer weiteren Verarbeitung eines aus dem Rückgewinnungsreaktors 10 erhaltenen Stroms gewonnen wird. Unter thermischer partieller Oxidation wird hier und nachfolgend die partielle Oxidation ohne katalytisches Medium verstanden. Die thermische partielle Oxidation kann daher auch als nichtkatalytische partielle Oxidation bezeichnet werden. Bevorzugt findet die thermische partielle Oxidation bei einer Temperatur von mindestens 1200° C statt. Schlussendlich werden das Kohlenstoffdioxid und der Wasserstoff wieder der Methanolsynthese zugeführt. Durch die Umwandlung des Methans in dem Rückgewinnungsreaktor 10 verbleiben als Reststoffe neben Kohlenstoffoxiden in wesentlicher Menge nur noch Stickstoff und Argon und damit genau diejenigen Stoffe, deren Entfernung aus dem Synthesekreislauf gewünscht ist.

Bezüglich des Rückgewinnungsstroms 9 ist bevorzugt, dass er - bei Zuführung zu der Methanolreaktoranordnung 3 - einen molaren Anteil an Wasserstoff von mindestens 70 % und/oder einen molaren Anteil an Kohlenstoffdioxid von mindestens 20 % aufweist.

Grundsätzlich ist die Art und Weise, wie die umgewandelten Kohlenstoffoxide und der Wasserstoff in den Rückgewinnungsstrom 9 gelangen oder zu diesem Zweck weiterverarbeitet werden beliebig. Vorzugsweise wird ein aus dem Rückgewinnungsreaktor 10 gewonnener Produktstrom 11 mit Kohlenstoffmonoxid einer Shiftvorrichtung 12 der Rückgewinnungsanordnung 8 zum Umwandeln des Kohlenstoffmonoxids durch eine Wassergas-Shift-Reaktion in Kohlenstoffdioxid zugeführt. Vorzugsweise weist der Produktstrom 11 einen molaren Anteil an Kohlenstoffmonoxid von mindestens 20 % oder von mindestens 25 % auf. Der molare Anteil an Kohlenstoffdioxid im Produktstrom 11 hingegen beträgt hingegen höchstens 15 % oder höchstens 10 %.

Grundsätzlich kann eine beliebige Menge oder ein beliebiger Anteil des Kohlenstoffmonoxids des Produktstroms 11 in der Shiftvorrichtung 12 in Kohlenstoffdioxid umgewandelt werden. Bevorzugt ist, dass ein molarer Anteil an Kohlenstoffmonoxid im Produktstrom 11 durch die Wassergas-Shift-Reaktion um mindestens 90 % verringert wird. So ist bevorzugt, dass der molare Anteil an Kohlenstoffmonoxid im Produktstrom 11 nach der Wassergas-Shift-Reaktion höchstens 1 % beträgt. Der molare Anteil an Kohlenstoffdioxid im Produktstrom 11 nach der Wassergas-Shift-Reaktion hingegen beträgt vorzugsweise mindestens 20 %. Kohlenstoffdioxid lässt sich leichter als Kohlenstoffmonoxid aus einem Prozessstrom entfernen, sodass die Rückgewinnung des Kohlenstoffs in Form von Kohlenstoffdioxid für die Methanolsynthese erleichtert wird.

Die Rückgewinnungsanordnung 8 kann grundsätzlich beliebige weitere Komponenten umfassen. Vorzugsweise weist die Rückgewinnungsanordnung 8 eine Kohlenstoffdioxidwaschvorrichtung 13 zum Auswaschen von Kohlenstoffdioxid auf und zumindest ein Teil des ausgewaschenen Kohlenstoffdioxids, vorzugsweise im Wesentlichen das gesamte ausgewaschene Kohlenstoffdioxid, ist von dem Rückgewinnungsstrom 9 umfasst. Insbesondere kann es sein, dass die Kohlenstoffdioxidwaschvorrichtung 13 zum Auswaschen von Kohlenstoffdioxid aus dem Produktstrom 11 eingerichtet ist. Vorzugsweise ist die Kohlenstoffdioxidwaschvorrichtung 13 der Shiftvorrichtung 12 prozesstechnisch nachgelagert. Grundsätzlich kann die Kohlenstoffdioxidwaschvorrichtung 13 das Kohlenstoffdioxid auf beliebige Weise auswaschen. Hier ist es bevorzugt, dass die Kohlenstoffdioxidwaschvorrichtung 13 einen Kaltmethanolkreislauf (cold methanol loop - CML) 14 zum Auswaschen des Kohlenstoffdioxids aufweist. Es ist bevorzugt, dass ein kohlenstoffdioxidhaltiger Strom 15, welcher insbesondere überwiegend aus Kohlenstoffdioxid besteht, aus der Kohlenstoffdioxidwaschvorrichtung 13 gewonnen wird. Vorzugsweise beträgt der molare Anteil an Kohlenstoffdioxid im kohlenstoffdioxidhaltigen Strom mindestens 50 %, insbesondere mindestens 90 %.

Dieser kohlenstoffdioxidhaltige Strom 15 umfasst das ausgewaschene Kohlenstoffdioxid. Das Entfernen des Kohlenstoffdioxids - und durch die zuvor erfolgte Umwandlung des Kohlenstoffmonoxids also der Kohlenstoffoxide insgesamt - erleichtert eine nachfolgende Rückgewinnung des Wasserstoffs bei gleichzeitiger Abtrennung der Reststoffe Stickstoff und Argon aus dem Produktstrom 11, welche nachfolgend beschrieben wird.

Bevorzugt ist weiter, dass ein weiterer Restgasstrom 16 aus der Kohlenstoffdioxidwaschvorrichtung 13 gewonnen und einer Wasserstoffrückgewinnungsanordnung 17 zum Gewinnen eines weiteren wasserstoffhaltigen Stroms 18a, b und eines Abgasstroms 19 zugeführt wird. Hier ist es bevorzugt, dass ein molarer Anteil von Wasserstoff in dem wasserstoffhaltigen Strom 18a, b höher ist als in dem Restgasstrom 16. Vorzugsweise umfasst der weitere Restgasstrom 16 einen molaren Anteil an Wasserstoff von mindestens 90 % und/oder einen molaren Anteil an Stickstoff von mindestens 3 %. Der weitere wasserstoffhaltige Strom 18a, b besteht vorzugsweise im Wesentlichen aus Wasserstoff.

Der Abgasstrom 19 umfasst vorzugsweise Stickstoff und Argon. Der Abgasstrom 19 kann ferner Restmengen von Wasserstoff, Methan und Kohlenstoffoxiden aufweisen. Bevorzugt ist, dass ein molarer Anteil von mindestens 30 % des Abgasstroms 19 durch Stickstoff und Argon gebildet wird. Insbesondere kann es sein, dass der molare Anteil von Stickstoff in dem Abgasstrom 19 mindestens 30 % beträgt.

Weiter ist bevorzugt, dass zumindest ein Teil des Wasserstoffs in dem wasserstoffhaltigen Strom 18a, b von dem Rückgewinnungsstrom 9 umfasst ist. Das kann insbesondere dadurch erfolgen, dass der Rückgewinnungsstroms 9 zumindest teilweise durch den wasserstoffhaltigen Strom 18a, b gebildet wird.

Grundsätzlich kann die Wasserstoffrückgewinnungsanordnung 17 auf beliebige Art und Weise ausgestaltet sein. Bevorzugt ist, dass die Wasserstoffrückgewinnungsanordnung 17 eine Rückgewinnungs-Druckwechsel-Adsorptionsvorrichtung (PSA) 20a, b zum Erhalt des weiteren wasserstoffhaltigen Stroms 18a, b und des Abgasstroms 19 aufweist.

Es ist bevorzugt, dass die Wasserstoffrückgewinnungsanordnung 17 nur eine Rückgewinnungs-Druckwechsel-Adsorptionsvorrichtung 20a, b aufweist. Zur weiteren Minimierung von Wasserstoffverlusten ist es aber vorteilhaft, dass die Wasserstoffrückgewinnungsanordnung 17 eine Vielzahl von prozesstechnisch hintereinander geschalteten Rückgewinnungs-Druckwechsel-Adsorptionsvorrichtungen (PSA) 20a, b zum Erhalt jeweiliger weiterer wasserstoffhaltiger Ströme 18a, b und des Abgasstroms 19 aufweist. Vorzugsweise bestehen die weiteren wasserstoffhaltigen Ströme 18a, b im Wesentlichen aus Wasserstoff.

Grundsätzlich kann der Synthesegasstrom 2 einen beliebigen Ursprung haben. Bevorzugt ist, dass einem Synthesegasreaktor 21 ein kohlenstoffhaltiger Energieträgerstrom 22 zum Gewinnen des Synthesegasstroms 2 zugeführt wird. Insbesondere kann es sein, dass der kohlenstoffhaltige Energieträgerstrom 22 einen Erdgasstrom 23 umfasst oder im Wesentlichen aus dem Erdgasstrom 23 besteht. Insbesondere kann es sein, dass die vorschlagsgemäße Anlage den Synthesegasreaktor 21 umfasst.

Bevorzugt ist, dass der Synthesegasstrom 2 vor Zuführung zu dem Synthesegasreaktor 21 einer Entschwefelungs- und Sättigungsanordnung 38 sowie ei-

nem Preformer 39 zugeführt wird, welche von der vorschlagsgemäßen Anlage umfasst sind.

Regelmäßig ist es vorteilhaft oder erforderlich, den Energieträgerstrom 22 vor der Zuführung zu dem Synthesegasreaktor 21 zu erhitzen. Grundsätzlich kann dies auf beliebige Art und Weise erfolgen. Es ist bevorzugt, dass der Energieträgerstrom 22 vor Zuführung zu dem Synthesegasreaktor 21 durch eine elektrisch betriebene Heizanordnung 24 vorerhitzt wird. Im Gegensatz zu einer Erhitzung in Erdgas befeuerten Wärmetauschern wird auf diese Weise kein Kohlenstoffdioxid freigesetzt. Es ist weiter bevorzugt, dass die Heizanordnung 24 durch klimaneutral gewonnenen Strom betrieben wird. Vorzugsweise umfasst die vorschlagsgemäße Anlage die Heizanordnung 24. Die Heizanordnung 24 kann aus einer einzelnen Heizvorrichtung bestehen. Die Heizanordnung 24 kann aber, wie in der Fig. 1 dargestellt, ebenso eine Vielzahl von Heizvorrichtungen umfassen. Diese Heizvorrichtungen der Heizanordnung 24 können jeweils prozesstechnisch vor der Entschwefelungs- und Sättigungsanordnung 38, dem Preformer 39 sowie dem Synthesegasreaktor 21 angeordnet sein.

Jede einzelne der dargestellten Heizvorrichtungen kann wiederum ihrerseits aus mehreren einzelnen Vorrichtungen bestehen. So ist es weiter bevorzugt, dass mindestens eine Heizvorrichtung der Heizanordnung 24 aus einer Kaskade von Einzel-Heizgeräten besteht. Dies ist deshalb vorteilhaft, weil die durch ein einzelnes elektrisch betriebenes Heizgerät erreichbare Temperaturerhöhung regelmäßig auf einen Wert begrenzt ist, welcher kleiner ist als die insgesamt angestrebte Temperaturerhöhung.

Bei dem Synthesegasreaktor 21 kann es sich grundsätzlich um eine beliebige Art von Reaktor zur Erzeugung von Synthesegas handeln. Bevorzugt ist, dass in dem Synthesegasreaktor 21 der Synthesegasstrom 2 durch eine autotherme Reformierung gewonnen wird, bei welcher eine katalytische partielle Oxidation die für die endothermen Dampfreformierungsreaktionen erforderliche Wärme bereitstellt. Weiter ist bevorzugt, dass der in dem Synthesegasreaktor 21 gewonnene Synthesegasstrom 2 ein molares Verhältnis, gegeben durch

$$S = \frac{n(H_2) - n(CO_2)}{n(CO) + n(CO_2)}$$

, von S < 2 aufweist. Insbesondere kann es sein, dass das molare Verhältnis S < 1,8 ist. Der Synthesegasstrom 2 ist dann unterstöchiometrisch und weist entsprechend für die Methanolsynthese einen Mangel an Wasserstoff auf.

Speziell bei der Synthesegaserzeugung durch autotherme Reformierung wird der Synthesegasstrom 2 mit einem niedrigeren Druck als in der Methanolsynthese benötigt bereitgestellt. Daher ist es bevorzugt, dass der Synthesegasstrom 2 vor Zuführung zu der Methanolreaktoranordnung 3 durch einen Synthesegaskompressor 25 druckerhöht wird. Vorzugsweise wird der Synthesegasstrom 2 vor Zuführung zu der Methanolreaktoranordnung 3 durch den Synthesegaskompressor 25 auf einen Druck von mindestens 70 bara druckerhöht.

Eine erste bevorzugte Möglichkeit besteht darin, dass der Synthesegaskompressor 25 elektrisch und insbesondere durch klimaneutral gewonnenen Strom betrieben wird. Klimaneutral gewonnener Strom wird vorzugsweise durch Kernkraft und/oder aus regenerativen Energiequellen gewonnen. Auf diese Weise kann der Betrieb des Synthesegaskompressors 25 also erfolgen, ohne dass Dampf für eine Dampfturbine durch Verfeuerung von Purgegas erzeugt werden müsste.

Ein klimaneutraler Antrieb ist aber auch beim Einsatz einer Dampfturbine zum Antrieb des Synthesegaskompressors 25 möglich. So sieht eine zweite bevorzugte Möglichkeit nämlich vor, dass der Synthesegaskompressor 25 durch eine mit Hochdruckdampf betrieben Dampfturbine angetrieben wird, wobei vorzugsweise der Hochdruckdampf elektrisch und insbesondere durch klimaneutral gewonnenen Strom überhitzt wird. Bevorzugt ist, dass der Hochdruckdampf im Prozess der Methanolsynthese anfällt.

Die Wasserstoffabtrennungsanordnung 5 kann grundsätzlich auf beliebige Art verwirklicht sein. Bevorzugt ist, dass die Wasserstoffabtrennungsanordnung 5 eine Abtrennungs-Druckwechsel-Adsorptionsvorrichtung (PSA) 26 zum Erhalt des wasserstoffhaltigen Stroms 6 und des Purgegasstroms 7 aufweist. Insbesondere kann es sein, dass der wasserstoffhaltige Strom 6 im Wesentlichen aus Wasserstoff besteht.

Grundsätzlich kann die Methanolreaktoranordnung 3 beliebig viele einzelne Methanolreaktoren unterschiedlichen Typs umfassen, welche wiederum prozesstechnisch grundsätzlich beliebig parallel oder in Reihe angeordnet sein können. Bevorzugt ist, dass die Methanolreaktoranordnung 3 einen ersten Methanolreaktor 27a zur Synthese von Methanol 1, einen dem ersten Methanolreaktor 27a prozesstechnisch nachgelagerten zweiten Methanolreaktor 27b zur Synthese von Methanol 1 sowie eine prozesstechnisch zwischen dem ersten Methanolreaktor 27a und dem zweiten Methanolreaktor 27b angeordnete Zwischenkondensationsstufe 28a zum Abtrennen von Rohmethanol 29 und zum Erhalt des Restgasstroms 4 aufweist. Das Rohmethanol 29 bildet die Grundlage zum Erhalten des Methanols 1. Speziell wird das Methanol 1 von dem Rohmethanol 29 umfasst. Bevorzugt ist, dass es sich bei dem ersten Methanolreaktor 27a und/oder bei dem zweiten Methanolreaktor 27b um einen isothermen Reaktor handelt.

Vorzugsweise umfasst die Methanolreaktoranordnung 3 auch eine dem zweiten Methanolreaktor 27b prozesstechnisch nachgelagerte weitere Zwischenkondensationsstufe 28b zum Abtrennen von Rohmethanol 29 und zum Erhalt eines Recyclegasstroms 30 mit unreagierten Restgasen.

Bevorzugt ist, dass die unreagierten Restgase des Recyclegasstroms 30 wieder der Methanolreaktoranordnung 3 und speziell dem ersten Methanolreaktor 27a zugeführt werden. Dabei kann es sein, dass der Recyclegasstrom 30 durch einen Recyclekompressor 34, welcher vorzugsweise von der vorschlagsgemäßen Anlage umfasst ist, druckerhöht wird. Der druckerhöhte Recyclegasstrom 30 kann, insbesondere dem Synthesegaskompressor 25 prozesstechnisch nachgelagert, mit dem Synthesegasstrom 2 zusammengeführt werden.

Bevorzugt ist, dass das Rohmethanol 29 aus der ersten Zwischenkondensationsstufe 28a und der zweiten Zwischenkondensationsstufe 28b einer Trennanordnung 37, vorzugsweise umfassend eine Entspannungstrommel 31 sowie eine Destillationsvorrichtung 32, zum Gewinnen von Methanol 1 zugeführt wird. Die Trennanordnung 37 ist von der Methanolreaktoranordnung 3 umfasst. Aus der Trennanordnung 37 und speziell der Entspannungstrommel 31 sowie der Destillationsvorrichtung 32 wird vorzugsweise ein jeweiliger Offgasstrom 33a, b erhalten, welcher dem Purgegasstrom 7 zugeführt wird.

Wie bereits oben beschrieben wurde, soll der wasserstoffhaltige Strom 6 der Methanolreaktoranordnung 3 zugeführt werden. Das kann auf unterschiedliche Weise erfolgen. Gemäß einer ersten bevorzugten Variante, welche in der Fig. 1 dargestellt ist, wird der Recyclegasstrom 30 mit dem wasserstoffhaltigen Strom 6 zusammengeführt. Vorzugsweise erfolgt dies dem Recyclekompressor 34 prozesstechnisch vorgelagert. Eine zweite bevorzugte Variante sieht vor, dass der wasserstoffhaltige Strom 6 mit dem Synthesegasstrom 2 zusammengeführt wird. Bevorzugt ist, dass das dem Synthesegaskompressor 25 prozesstechnisch vorgelagert erfolgt. Durch die Wahl der Variante ergibt sich eine jeweils unterschiedliche Dimensionierung der genannten Kompressoren.

Vorzugsweise umfasst die vorschlagsgemäße Anlage den Recyclekompressor 34. Dabei erfolgt die Zuführung des Recyclegasstroms 30 mit dem wasserstoffhaltigen Strom 6 zu dem Synthesegasstrom 2 bevorzugt dem Synthesegaskompressor 25 prozesstechnisch nachgelagert.

Speziell bei einer Erzeugung des Synthesegasstroms 2 durch autotherme Reformierung ist das Synthesegas des Synthesegasstroms 2 unterstöchiometrisch, weist also einen Mangel an Wasserstoff auf. Dem kann durch Zufuhr von Wasserstoff aus einer Quelle von außerhalb des Synthesekreislaufs begegnet werden. Dementsprechend ist es bevorzugt, dass der Rückgewinnungsanordnung 8 ein Wasserstoffstrom 35 zugeführt wird, wobei der Wasserstoff des Rückgewinnungsstroms 9 zumindest teilweise von dem Wasserstoffstrom 35 stammt. Weiter ist es bevorzugt, dass der Wasserstoffstrom 35 aus einer Wasserelektrolyse gewonnen wird, wobei es insbesondere sein kann, dass die Wasserelektrolyse durch klimaneutral gewonnenen Strom gespeist wird. Der Wasserstoffstrom 35 muss dabei nicht vollständig aus Wasserstoff bestehen. Es reicht, dass der Wasserstoffstrom 35 überwiegend oder im Wesentlichen aus Wasserstoff besteht.

Bevorzugt ist weiter, dass der Rückgewinnungsstrom 9 einem Rückgewinnungsstromkompressor 36 der Rückgewinnungsanordnung 8 zur Druckerhöhung zugeführt wird. Dies bietet sich insbesondere deshalb an, weil Wasserstoff aus einer Wasserelektrolyse nur mit geringem Druck gewonnen wird. Daher ist es ebenso bevorzugt, dass der Rückgewinnungskompressor 36 der Zuführung des Wasserstoffstroms 35 zur Rückgewinnungsanordnung 8 prozesstechnisch nachgelagert ist.

Die vorschlagsgemäße Anlage dient der Synthese von Methanol 1 und umfasst die Methanolreaktoranordnung 3 zur Synthese von Methanol 1, welcher Methanolreaktoranordnung 3 der Synthesegasstrom 2 mit Wasserstoff und Kohlenstoffoxiden zugeführt wird.

Die vorschlagsgemäße Anlage weist ebenso die Wasserstoffabtrennungsanordnung 5 zum Erhalt des wasserstoffhaltigen Stroms 6 auf, welcher Wasserstoffabtrennungsanordnung 5 der aus der Methanolreaktoranordnung 3 gewonnene Restgasstrom 4 mit unreagiertem Wasserstoff und unreagierten Kohlenstoffoxiden zugeführt wird, wobei der wasserstoffhaltige Strom 6 der Methanolreaktoranordnung 3 zugeführt wird und einen höheren molaren Wasserstoffanteil als der Restgasstrom 4 aufweist und wobei der Purgegasstrom 7 mit Methan aus der Wasserstoffabtrennungsanordnung 5 gewonnen wird.

Die vorschlagsgemäße Anlage ist dadurch gekennzeichnet, dass die Anlage die Rückgewinnungsanordnung 8 zum Gewinnen des Rückgewinnungsstroms 9 mit Kohlenstoffdioxid und Wasserstoff aufweist, welcher Rückgewinnungsanordnung 8 der Purgegasstrom 7 zugeführt wird, dass die Rückgewinnungsanordnung 8 den Rückgewinnungsreaktor 10 zum zumindest teilweisen Umwandeln des Methans des Purgegasstroms 7 in Kohlenstoffoxide und Wasserstoff durch thermische partielle Oxidation aufweist und dass der Rückgewinnungsstrom 8 der Methanolreaktoranordnung 3 zugeführt wird.

Die vorschlagsgemäße Anlage ist zur Ausführung des vorschlagsgemäßen Verfahrens eingerichtet.

**Patentansprüche**

1. Verfahren zur Synthese von Methanol (1), wobei ein Synthesegasstrom (2) mit Wasserstoff und Kohlenstoffoxiden einer Methanolreaktoranordnung (3) zur Synthese von Methanol (1) zugeführt wird, wobei ein Restgasstrom (4) mit unreagiertem Wasserstoff und unreagierten Kohlenstoffoxiden aus der Methanolreaktoranordnung (3) gewonnen wird, wobei der Restgasstrom (4) einer Wasserstoffabtrennungsanordnung (5) zum Erhalt eines wasserstoffhaltigen Stroms (6) zugeführt, wobei der wasserstoffhaltige Strom (6) der Methanolreaktoranordnung (3) zugeführt wird und einen höheren molaren Wasserstoffanteil als der Restgasstrom (4) aufweist und wobei ein Purgegasstrom (7) mit Methan aus der Wasserstoffabtrennungsanordnung (5) gewonnen wird, **dadurch gekennzeichnet, dass** der Purgegasstrom

(7) einer Rückgewinnungsanordnung (8) zum Gewinnen eines Rückgewinnungsstroms (9) mit Kohlenstoffdioxid und Wasserstoff zugeführt wird, dass die Rückgewinnungsanordnung (8) einen Rückgewinnungsreaktor (10) zum zumindest teilweisen Umwandeln des Methans des Purgegasstroms (7) in Kohlenstoffoxide und Wasserstoff durch thermische partielle Oxidation aufweist und dass der Rückgewinnungsstrom (9) der Methanolreaktoranordnung (3) zugeführt wird.

2. Verfahren nach Anspruch 1, **dadurch gekennzeichnet, dass** ein aus dem Rückgewinnungsreaktor (10) gewonnener Produktstrom (11) mit Kohlenstoffmonoxid einer Shiftvorrichtung (12) der Rückgewinnungsanordnung (8) zum Umwandeln des Kohlenstoffmonoxids durch eine Wassergas-Shift-Reaktion in Kohlenstoffdioxid zugeführt wird, vorzugsweise, dass ein molarer Anteil an Kohlenstoffmonoxid im Produktstrom (11) durch die Wassergas-Shift-Reaktion um mindestens 90 % verringert wird.

3. Verfahren nach Anspruch 1 oder 2, **dadurch gekennzeichnet, dass** die Rückgewinnungsanordnung (8) eine Kohlenstoffdioxidwaschvorrichtung (13) zum Auswaschen von Kohlenstoffdioxid, vorzugsweise aus dem Produktstrom (11), aufweist und dass zumindest ein Teil des ausgewaschenen Kohlenstoffdioxids von dem Rückgewinnungsstrom (9) umfasst ist.

4. Verfahren nach Anspruch 3, **dadurch gekennzeichnet** insbesondere, dass ein weiterer Restgasstrom (16) aus der Kohlenstoffdioxidwaschvorrichtung (13) gewonnen wird und einer Wasserstoffrückgewinnungsanordnung (17) zum Gewinnen eines weiteren wasserstoffhaltigen Stroms (18a, b) und eines Abgasstroms (19), welcher vorzugsweise Stickstoff und Argon umfasst, zugeführt wird, insbesondere, dass zumindest ein Teil des Wasserstoffs in dem wasserstoffhaltigen Strom (18a, b) von dem Rückgewinnungsstrom (9) umfasst ist.

5. Verfahren nach Anspruch 4, **dadurch gekennzeichnet, dass** die Wasserstoffrückgewinnungsanordnung (17) eine Rückgewinnungs-Druckwechsel-Adsorptionsvorrichtung (PSA) (20a, b) zum Erhalt des weiteren wasserstoffhaltigen Stroms (18a, b) und des Abgasstroms (19) aufweist, vorzugsweise, dass der weitere wasserstoffhaltige Strom (18a, b) im Wesentlichen aus Wasserstoff besteht.

6. Verfahren nach Anspruch 5, **dadurch gekennzeichnet, dass** die Wasserstoffrückgewinnungsanordnung (17) eine Vielzahl von prozesstechnisch hintereinander geschalteten Rückgewinnungs-Druckwechsel-Adsorptionsvorrichtungen (PSA)

(20a, b) zum Erhalt jeweiliger weiterer wasserstoffhaltiger Ströme (18a, b) und des Abgasstroms (19) aufweist, vorzugsweise, dass die weiteren wasserstoffhaltigen Ströme (18a, b) im Wesentlichen aus Wasserstoff bestehen.

7. Verfahren nach einem der Ansprüche 1 bis 6, **dadurch gekennzeichnet, dass** einem Synthesegasreaktor (21) ein kohlenstoffhaltiger Energieträgerstrom (22) zum Gewinnen des Synthesegasstroms (2) zugeführt wird, insbesondere, dass der kohlenstoffhaltige Energieträgerstrom (22) einen Erdgasstrom (23) umfasst, vorzugsweise im Wesentlichen aus dem Erdgasstrom (23) besteht.

8. Verfahren nach Anspruch 7, **dadurch gekennzeichnet, dass** der Energieträgerstrom (22) vor Zuführung zu dem Synthesegasreaktor (21) durch eine elektrisch betriebene Heizanordnung (24) vorerhitzt wird, vorzugsweise, dass die Heizanordnung (24) durch klimaneutral gewonnenen Strom betrieben wird.

9. Verfahren nach Anspruch 7 oder 8, **dadurch gekennzeichnet, dass** in dem Synthesegasreaktor (21) der Synthesegasstrom (2) durch eine autotherme Reformierung gewonnen wird, bei welcher autothermen Reformierung eine katalytische partielle Oxidation die für die endothermen Dampfreformierungsreaktionen erforderliche Wärme bereitstellt, vorzugsweise, dass der in dem Synthesegasreaktor (21) gewonnene Synthesegasstrom (2) ein molares

$$S = \frac{n(H_2) - n(CO_2)}{n(CO) + n(CO_2)}$$

Verhältnis, gegeben durch von S < 2, insbesondere von S < 1,8, aufweist.

10. Verfahren nach einem der Ansprüche 1 bis 9, **dadurch gekennzeichnet, dass** der Synthesegasstrom (2) vor Zuführung zu der Methanolreaktoranordnung (3) durch einen Synthesegaskompressor (25) druckerhöht wird, vorzugsweise, dass der Synthesegaskompressor (25) elektrisch, insbesondere durch klimaneutral gewonnenen Strom betrieben wird.

11. Verfahren nach Anspruch 10, **dadurch gekennzeichnet, dass** der Synthesegaskompressor (25) durch eine mit Hochdruckdampf betrieben Dampfturbine angetrieben wird, vorzugsweise, dass der Hochdruckdampf elektrisch, insbesondere durch klimaneutral gewonnenen Strom, überhitzt wird.

12. Verfahren nach einem der Ansprüche 1 bis 11, **dadurch gekennzeichnet, dass** die Wasserstoffabtrennungsanordnung (5) eine Abtrennungs-Druckwechsel-Adsorptionsvorrichtung (PSA) (26) zum Erhalt des wasserstoffhaltigen Stroms (6) und des Purgegasstroms (7) aufweist, vorzugsweise, dass der wasserstoffhaltige Strom (6) im Wesentlichen aus Wasserstoff besteht.

13. Verfahren nach einem der Ansprüche 1 bis 12, **dadurch gekennzeichnet, dass** die Methanolreaktoranordnung (3) einen ersten Methanolreaktor (27a) zur Synthese von Methanol (1), einen dem ersten Methanolreaktor (27a) prozesstechnisch nachgelagerten zweiten Methanolreaktor (27b) zur Synthese von Methanol (1) sowie eine prozesstechnisch zwischen dem ersten Methanolreaktor (27a) und dem zweiten Methanolreaktor (27b) angeordnete Zwischenkondensationsstufe (28a) zum Abtrennen von Rohmethanol (29) und zum Erhalt des Restgasstroms (4) aufweist.

14. Verfahren nach einem der Ansprüche 1 bis 13, **dadurch gekennzeichnet, dass** der Rückgewinnungsanordnung (8) ein Wasserstoffstrom (35) zugeführt wird, wobei der Wasserstoff des Rückgewinnungsstroms (9) zumindest teilweise von dem Wasserstoffstrom (35) stammt, vorzugsweise, dass der Wasserstoffstrom (35) aus einer Wasserelektrolyse gewonnen wird, insbesondere, dass die Wasserelektrolyse durch klimaneutral gewonnenen Strom gespeist wird.

15. Anlage zur Synthese von Methanol (1) mit einer Methanolreaktoranordnung (3) zur Synthese von Methanol (1), welcher Methanolreaktoranordnung (3) ein Synthesegasstrom (2) mit Wasserstoff und Kohlenstoffoxiden zugeführt wird, mit einer Wasserstoffabtrennungsanordnung (5) zum Erhalt eines wasserstoffhaltigen Stroms (6), welcher Wasserstoffabtrennungsanordnung (5) ein aus der Methanolreaktoranordnung (3) gewonnener Restgasstrom (4) mit unreagiertem Wasserstoff und unreagierten Kohlenstoffoxiden zugeführt wird, wobei der wasserstoffhaltige Strom (6) der Methanolreaktoranordnung (3) zugeführt wird und einen höheren molaren Wasserstoffanteil als der Restgasstrom (4) aufweist und wobei ein Purgegasstrom (7) mit Methan aus der Wasserstoffabtrennungsanordnung (5) gewonnen wird, **dadurch gekennzeichnet, dass** die Anlage eine Rückgewinnungsanordnung (8) zum Gewinnen eines Rückgewinnungsstroms (9) mit Kohlenstoffdioxid und Wasserstoff aufweist, welcher Rückgewinnungsanordnung (8) der Purgegasstrom (7) zugeführt wird, dass die Rückgewinnungsanordnung (8) einen Rückgewinnungsreaktor (10) zum zumindest teilweisen Umwandeln des Methans des Purgegasstroms (7) in Kohlenstoffoxide und Wasserstoff durch thermische partielle Oxidation aufweist und dass der Rückgewinnungsstrom (8) der Methanolreaktoranordnung (3) zugeführt wird.

Fig. 1

EP 4 212 502 A1

Europäisches
Patentamt

European
Patent Office

Office européen
des brevets

# EUROPÄISCHER RECHERCHENBERICHT

**Nummer der Anmeldung**

EP 22 15 1780

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 3 205 622 B1 (WAGNER ULRICH [DE]; BALTHASAR WOLFF [DE]; MUELLER DIERK [DE]) 9. Mai 2018 (2018-05-09) * das ganze Dokument * ----- | 1-15 | INV. C07C29/152 C07C31/04 C01B3/34 B01J8/00 |
| A | DK 2017 00695 A1 (TOPSOE HALDOR AS [DK]) 25. Juni 2019 (2019-06-25) * das ganze Dokument * ----- | 1-15 | |

**RECHERCHIERTE SACHGEBIETE (IPC)**

C07C
B01J
C01C
C01B

Der vorliegende Recherchenbericht wurde für alle Patentansprüche erstellt

| Recherchenort | Abschlußdatum der Recherche | Prüfer |
|---|---|---|
| Den Haag | 27. Juni 2022 | Fritz, Martin |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 22 15 1780

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-06-2022

| Im Recherchenbericht angeführtes Patentdokument | Datum der Veröffentlichung | Mitglied(er) der Patentfamilie | Datum der Veröffentlichung |
|---|---|---|---|
| EP 3205622 B1 | 09-05-2018 | CA 3013967 A1 | 17-08-2017 |
| | | CN 109071387 A | 21-12-2018 |
| | | DK 3205622 T3 | 06-08-2018 |
| | | EP 3205622 A1 | 16-08-2017 |
| | | JP 6716722 B2 | 01-07-2020 |
| | | JP 2019513816 A | 30-05-2019 |
| | | US 2019047931 A1 | 14-02-2019 |
| | | WO 2017137581 A1 | 17-08-2017 |
| DK 201700695 A1 | 25-06-2019 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3205622 B1